(19) Europäisches Patentamt
European Patent Office
Office européen des brevets

(11) Veröffentlichungsnummer: **0 297 490**
**A1**

# (12) EUROPÄISCHE PATENTANMELDUNG

(21) Anmeldenummer: **88110245.3**

(22) Anmeldetag: **28.06.88**

(51) Int. Cl.[4]: **C07D 487/04 , A01N 43/90 , //(C07D487/04,251:00,231:00)**

(30) Priorität: **01.07.87 DE 3722072**

(43) Veröffentlichungstag der Anmeldung: **04.01.89 Patentblatt 89/01**

(84) Benannte Vertragsstaaten: **AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Krüger, Martin Dr.**
**Schluchseestrasse 65**
**D-1000 Berlin 28(DE)**
Erfinder: **Westermann, Jürgen Dr.**
**Elberfelderstrasse 19**
**D-1000 Berlin 21(DE)**
Erfinder: **Arndt, Friedrich Dr.**
**Mühlenfeldstrasse 10**
**D-1000 Berlin 28(DE)**
Erfinder: **Rees, Richard Dr.**
**Speerweg 8**
**D-1000 Berlin 28(DE)**
Erfinder: **Hunt, Russel George Dr.**
**45 The Limes, Harston**
**Cambridge(GB)**

(54) **6,7-Dihydro-pyrazolo(1,5-a) (1,3,5)triazin-2-sulfonsäureamide, Verfahren zu ihrer Herstellung und ihre Verwendung als Mittel mit herbizider und pflanzenwuchsregulierender Wirkung.**

(57) Die Erfindung betrifft neue 6,7-Dihydro-pyrazolo[1,5-a][1,3,5]triazin-2-sulfonsäureamide der allgemeinen Formel I

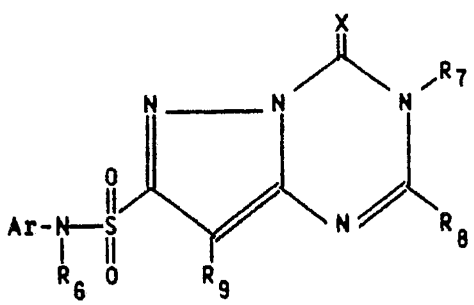

(I) ,

in der Ar, $R_6$, $R_7$, $R_8$, $R_9$ und X die in der Beschreibung genannten Bedeutungen haben, Verfahren zu ihrer Herstellung und ihre Verwendung als Mittel mit herbizider und pflanzenwuchsregulierender Wirkung.

EP 0 297 490 A1

# 6,7-DIHYDRO-PYRAZOLO[1,5-a][1,3,5]TRIAZIN-2-SULFONSÄUREAMIDE, VERFAHREN ZU IHRER HERSTELLUNG UND IHRE VERWENDUNG ALS MITTEL MIT HERBIZIDER UND PFLANZENWUCHSREGULIERENDER WIRKUNG

Die Erfindung betrifft neue 6,7-Dihydro-pyrazolo[1,5-a][1,3,5]triazin-2-sulfonsäureamide, Verfahren zu ihrer Herstellung und ihre Verwendung als Mittel mit herbizider und pflanzenwuchsregulierender Wirkung.

Es ist bereits bekannt, daß Triazolo-pyrimidin-sulfonamide eine herbizide Wirkung besitzen (EP-Anmeldungen 0 142 152 und 0 150 974). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

Aufgabe der vorliegenden Erfindung ist die Bereitstellung von neuen Verbindungen, die diese Nachteile nicht aufweisen und in ihren biologischen Eigenschaften den bisher bekannten Verbindungen überlegen sind.

Es wurde nun gefunden, daß 6,7-Dihydro-pyrazolo[1,5-a][1,3,5]triazin-2-sulfonsäureamide der allgemeinen Formel I

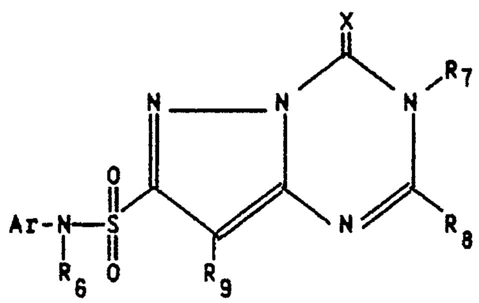

(I) ,

in der

Ar eine Phenyl-, Naphthyl-, Pyridyl- oder Thienylgruppe der allgemeinen Formeln

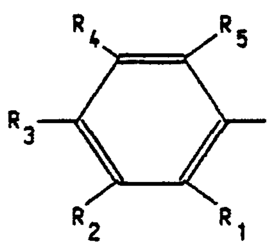
,
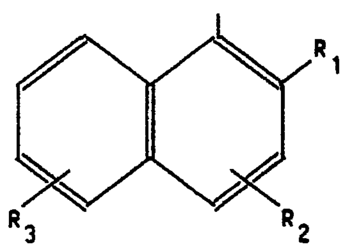
,
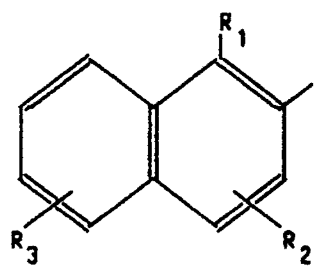
,

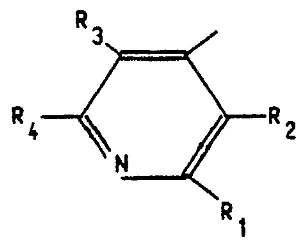, 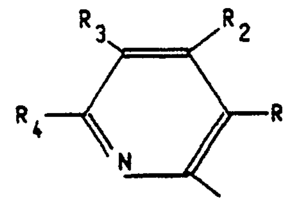, 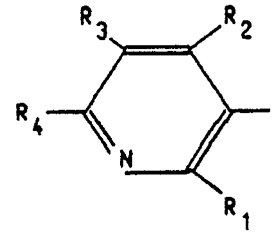,

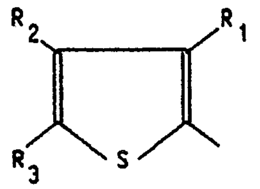 und 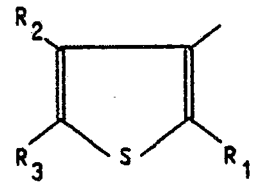,

$R_1$, $R_2$, $R_3$, $R_4$ und $R_5$, die gleich oder verschieden sind, ein Wasserstoffatom, einen gegebenenfalls ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substituierten $C_1$-$C_6$-Alkyl-, $C_2$-$C_6$-Alkenyl- oder $C_2$-$C_6$-Alkinylrest, ein Halogenatom, eine $C_1$-$C_4$-Alkoxygruppe, eine Gruppe $R_{10}$-O-CO-, eine Aminocarbonylgruppe $R_{11}R_{12}$N-CO-, eine Aminogruppe $R_{11}R_{12}$N-, eine Cyanogruppe, eine Nitrogruppe, eine Schwefel enthaltende Gruppe $R_{10}$-S(O)$_n$-, eine Acylgruppe $R_{10}$-CO-, eine Gruppe $R_{10}$-O-CO-$(CH_2)_n$- oder einen durch $C_1$-$C_4$-Alkyl, Halogen oder Nitro substituierten Phenyl- oder Phenoxyrest,
$R_6$ ein Wasserstoffatom, eine Acylgruppe $R_{10}$-CO-, eine Gruppe $R_{10}$-O-CO-, eine $C_1$-$C_6$-Alkylgruppe, eine $C_2$-$C_6$-Alkenylgruppe, eine $C_2$-$C_6$-Alkinylgruppe, eine Phenyl-$C_1$-$C_4$-alkylgruppe, eine Aminocarbonylgruppe $R_{11}R_{12}$N-CO-, ein Alkalimetallatom, ein einwertiges Metalläquivalent aus der Gruppe der Erdalkalimetalle und weitere Metalle oder einen gegebenenfalls durch $C_1$-$C_6$-Alkyl substituierten Ammoniumrest,
$R_7$ und $R_8$, die gleich oder verschieden sind, ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest, einen durch $R_1$, $R_2$, $R_3$, $R_4$ und $R_5$ substituierten Phenylrest, eine Acylgruppe $R_{10}$-CO-, eine Gruppe $R_{10}$-O-CO-, eine Gruppe $R_{10}$-O-CO-$(CH_2)_n$-, eine Aminocarbonylgruppe $R_{11}R_{12}$N-CO-, oder eine Sulfonylgruppe $R_{10}$-$SO_2$-,
$R_9$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest, einen durch $R_1$, $R_2$, $R_3$, $R_4$ und $R_5$ substituierten Phenylrest, eine Acylgruppe $R_{10}$-CO-, eine Gruppe $R_{10}$-O-CO-, eine Gruppe $R_{10}$-O-CO-$(CH_2)_n$-, eine Aminocarbonylgruppe $R_{11}R_{12}$N-CO-, eine Sulfonylgruppe $R_{10}$-$SO_2$- oder $R_{10}$-O-$SO_2$-, eine Aminosulfonylgruppe $R_{11}R_{12}$N-$SO_2$-, eine Cyanogruppe oder eine Nitrogruppe,
$R_{10}$ ein Wasserstoffatom, eine gegebenenfalls ein- oder mehrfach, gleich oder verschieden durch Halogen, Hydroxy oder $C_1$-$C_4$-Alkoxy substituierte $C_1$-$C_6$-Alkyl-, $C_2$-$C_6$-Alkenyl-, $C_2$-$C_6$-Alkinyl- oder Phenyl-$C_1$-$C_4$-alkylgruppe oder eine gegebenenfalls durch Halogen, Nitro oder $C_1$-$C_4$-Alkyl substituierte Phenylgruppe,
$R_{11}$ und $R_{12}$, die gleich oder verschieden sind, ein Wasserstoffatom, einen gegebenenfalls ein- oder mehrfach, gleich oder verschieden durch Halogen, Hydroxy oder $C_1$-$C_4$-Alkoxy substituierten $C_1$-$C_6$-Alkyl-, $C_2$-$C_6$-Alkenyl-oder $C_2$-$C_6$-Alkinylrest oder
$R_{11}$ und $R_{12}$ gemeinsam mit dem benachbarten Stickstoffatom die Pyrrolidinyl-, Piperidino- oder Morpholinogruppe und
X ein Sauerstoff- oder Schwefelatom
n 0, 1 oder 2
bedeuten, eine interessante herbizide und pflanzenwuchsregulierende Wirkung zeigen.

Der Begriff "Halogen" im Zusammenhang mit Alkyl, Alkenyl, Alkinyl oder Phenyl bedeutet, daß ein oder mehrere Wasserstoffatome durch ein oder mehrere Halogenatome ersetzt sind.

Die Bezeichnung "Halogen" umfaßt Fluor, Chlor, Brom und Jod.

Als besonders wirksam haben sich solche 6,7-Dihydro-pyrazolo[1,5-a][1,3,5]triazin-2-sulfonsäureamide der allgemeinen Formel I erwiesen, bei denen

Ar eine Phenylgruppe der allgemeinen Formel

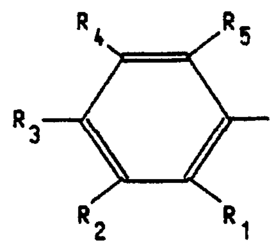

$R_1$ und $R_5$, die gleich oder verschieden sind, ein Halogenatom, eine Methylgruppe, eine Trifluoromethylgruppe, eine Nitrogruppe, eine Methoxygruppe oder eine Methoxycarbonylgruppe,
$R_2$, $R_3$ und $R_4$, die gleich oder verschieden sind, ein Wasserstofatom, ein Halogenatom, eine Trifluormethyl- oder eine $C_1$-$C_4$-Alkylgruppe,
$R_6$ ein Wasserstoffatom, ein einwertiges Metalläquivalent oder eine $C_1$-$C_4$-Acylgruppe,
$R_7$ und $R_8$, die gleich oder verschieden sind, ein Wasserstoffatom, eine $C_1$-$C_4$-Acylgruppe, eine $C_1$-$C_4$-Alkylgruppe oder eine $C_2$-$C_4$-Alkenylgruppe oder eine Phenylgruppe,
$R_9$ ein Wasserstoffatom, eine $C_1$-$C_4$-Acylgruppe, eine Gruppe $R_{10}$-O-CO-, eine Aminocarbonylgruppe $R_{11}R_{12}$N-CO-, eine Sulfonylgruppe $R_{10}$-$SO_2$-, eine Cyanogruppe oder eine Nitrogruppe und
X ein Sauerstoff- oder Schwefelatom bedeuten.

Die erfindungsgemäßen Verbindungen der allgemeinen Formel I können zum Beispiel hergestellt werden, indem man

A) Amine der allgemeinen Formel II,

Ar-NH-$R_6$ (II) ,

in der Ar die oben angegebene Bedeutung hat und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Sulfonsäurechlorid der allgemeinen Formel III

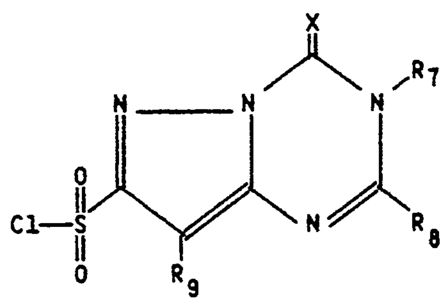

(III) ,

in der $R_7$, $R_8$, $R_9$ und X die oben angegebenen Bedeutungen haben, in einem geeigneten Lösungsmittel in Gegenwart eines Säureakzeptors umsetzt, oder

B) eine Verbindung der allgemeinen Formel IV

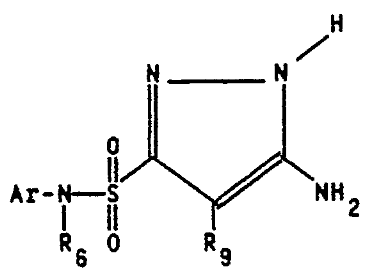

(IV) ,

in der Ar und $R_9$ die oben angegebenen Bedeutungen haben und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Isocyanat oder Isothiocyanat der Formel V

$R_7$-NCX (V),

in der $R_7$ und X die oben angegebenen Bedeutungen haben, in einem geeigneten Lösungsmittel gegebenenfalls in Gegenwart eines Säureakzeptors beziehungsweise Katalysators umsetzt, und die entstandenen Verbindungen der allgemeinen Formel VI

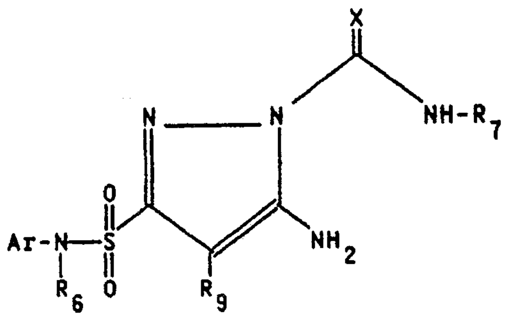

(VI),

in der Ar, $R_7$, $R_9$ und X die oben angegebenen Bedeutungen haben und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-C($OR_{10}$)$_3$ (VII),

in der $R_{10}$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl-oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest oder einen durch $R_1$, $R_2$, $R_3$, $R_4$ und $R_5$ substituierten Phenylrest bedeutet, in einem geeigneten Lösungsmittel, das auch der Orthoester selbst sein kann, umsetzt, oder

C) eine Verbindung der allgemeinen Formel IV

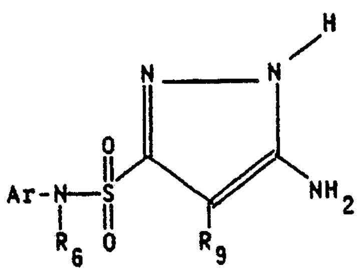

(IV),

in der Ar und $R_9$ die oben angegebenen Bedeutungen haben und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-C($OR_{10}$)$_3$ (VII),

in der $R_{10}$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl-oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, zu Verbindungen der allgemeinen Formel VIII

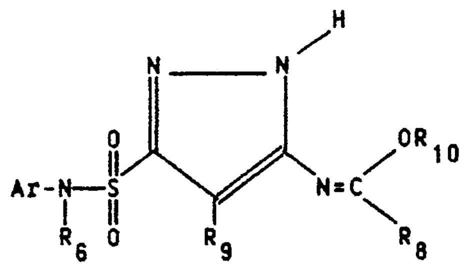

(VIII) ,

in der Ar, $R_9$ und $R_{10}$ die oben angegebenen Bedeutungen haben, $R_{10}$ aber nicht für ein Wasserstoffatom steht, $R_6$ ein Wasserstoffatom oder eine $C_1$-$C_6$-Alkyl-, $C_2$-$C_6$-Alkenyl- oder $C_2$-$C_6$-Alkinylgruppe und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest oder einen durch $R_1$, $R_2$, $R_3$, $R_4$ und $R_5$ substituierten Phenylrest bedeuten, in einem geeigneten Lösungsmittel, das auch der Orthoester selbst sein kann, umsetzt und dann die Verbindungen der allgemeinen Formel VIII mit einem Isocyanat oder Isothiocyanat der Formel V

$R_7$-NCX (V)

in der $R_7$ und X die oben angegebenen Bedeutungen haben, in einem geeigneten Lösungsmittel gegebenenfalls in Gegenwart eines Säureakzeptors beziehungsweise Katalysators umsetzt, oder

D) eine Verbindung der allgemeinen Formel IX

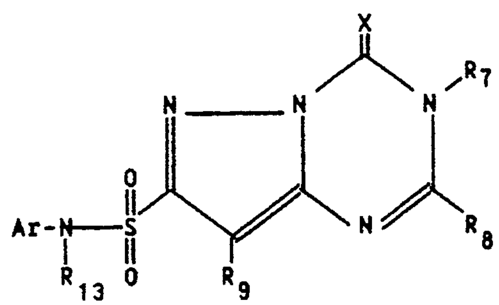

(IX) ,

in der Ar, $R_7$, $R_8$, $R_9$ und X die oben angegebenen Bedeutungen haben und $R_{13}$ ein Wasserstoffatom oder ein einwertiges Metalläquivalent bedeutet, mit Verbindungen der allgemeinen Formel X

$R_{10}$-Hal (X) ,

oder der allgemeinen Formel XI

$R_{10}$-CO-Hal (XI) ,

in denen $R_{10}$ die oben genannte Bedeutung hat, aber nicht für ein Wasserstoffatom steht, und Hal Chlor oder Brom bedeutet, oder der allgemeinen Formel XII

$R_{10}$-CO-O-CO-$R_{10}$ (XII) ,

in der $R_{10}$ die oben genannte Bedeutung hat, in einem geeigneten Lösungsmittel umsetzt, oder

E) eine Verbindung der allgemeinen Formel XIII

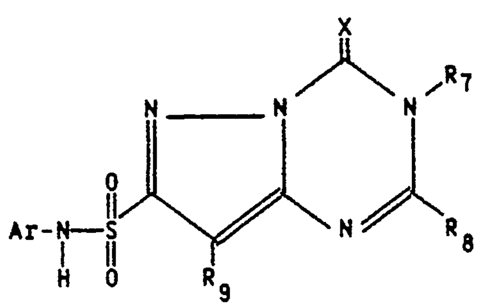

(XIII) ,

in der Ar, $R_7$, $R_8$, $R_9$ und X die oben angegebenen Bedeutungen haben, mit einer Verbindung der allgemeinen Formel XIV

M - Y (XIV),

in der M ein einwertiges Metalläquivalent und Y ein Wasserstoffatom, eine Hydroxy-, Niederalkyl-, Niederalkoxy- oder Aminogruppe bedeutet, in einem Lösungsmittel umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfone und Sulfoxide, wie zum Beispiel Dimethylsulfoxid und Sulfolan, und Basen, wie zum Beispiel Pyridin.

Die Reaktionen werden vorzugsweise zwischen Raumtemperatur und dem Siedepunkt des jeweiligen Reaktionsgemisches durchgeführt. Die Reaktionen lassen sich bei dem Druck der Umgebung durchführen, wenngleich sie auch bei erhöhtem beziehungsweise vermindertem Druck durchgeführt werden können.

Die Verfahrensvariante A) wird vorzugsweise in chlorierten Kohlenwasserstoffen wie Dichlormethan oder Dichlorethan, in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin und Pyridin. Pyridin kann sowohl als Katalysator als auch als Lösungsmittel bei dieser Reaktion verwendet werden.

Die Verfahrensvarianten B) und C) werden bevorzugt in Gegenwart von Verdünnungsmitteln wie aliphatischen, alicyclischen und aromatischen Kohlenwasserstoffen, die jeweils gegebenenfalls chloriert sein können, wie zum Beispiel Hexan, Cyclohexan, Petrolether, Ligroin, Benzol, Toluol, Xylol, Methylenchlo rid, Chloroform, Kohlenstofftetrachlorid, Ethylenchlorid und Trichlorethylen, Ethern, wie zum Beispiel Diisopropylether, Dibutylether, Propylenoxid, Dioxan und Tetrahydrofuran, Ketonen, wie zum Beispiel Aceton, Methylethylketon, Methylisopropylketon und Methylisobutylketon, Nitrilen, wie zum Beispiel Acetonitril und Propionitril, Estern, wie zum Beispiel Ethylacetat und Amylacetat, Säureamiden, wie zum Beispiel Dimethylformamid und Dimethylacetamid, Sulfonen und Sulfoxiden, wie zum Beispiel Dimethylsulfoxid und Sulfolan, und Basen, wie zum Beispiel Pyridin, und gegebenenfalls in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin oder Pyridin.

Die Verfahrensvariante D) wird vorzugsweise so durchgeführt, daß man eine Verbindung der allgemeinen Formel IX in einem geeigneten Lösungsmittel mit Verbindungen der allgemeinen Formeln X, XI oder XII umsetzt, die in der Regel schwer löslichen anorganischen Salze abfiltriert und die gewünschten Verbindungen nach Abdampfen der Lösungsmittel erhält.

Die Verfahrensvariante E) wird vorzugsweise so durchgeführt, daß man eine Verbindung der allgemeinen Formel XIII in einem geeigneten Lösungsmittel mit einer Metallbase, wie einem Metallhydroxid, Metallhydrid, Metallalkyl oder Metallamid, umsetzt und die in der Regel schwerer löslichen Salze abfiltriert oder nach Abdampfen der Lösungsmittel erhält.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels beinormalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Kristalle dar, die wenig löslich in Wasser und in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsufloxid, sind.

Die Sulfonsäurechloride der allgemeinen Formel III sind neu und lassen sich aus in der Literatur beschriebenen beziehungsweise nach bekannten Verfahren darstellbaren 2-Benzylthio-6,7-dihydro-pyrazolo-[1,5-a][1,3,5]triazin-7-onen der allgemeinen Formel XV

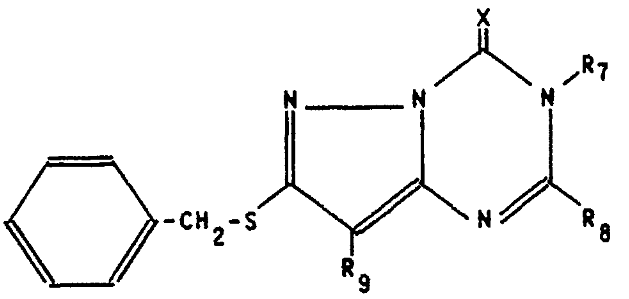

(XV) ,

in der $R_7$, $R_8$, $R_9$ und X die oben angegebenen Bedeutungen haben, durch Umsetzung mit Chlor in Wasser oder Wasser/Essigsäuregemischen herstellen.

Amine der allgemeinen Formel II, Isocyanate beziehungsweise Isothiocyanate der allgemeinen Formel V und Orthoester der allgemeinen Formel VII sind zum Teil käuflich oder nach bekannten beziehungsweise in der Literatur beschriebenen Verfahren darstellbar.

Die erfindungsgemäßen Wirkstoffe beeinflussen das Pflanzenwachstum und können deshalb als Wachstumsregulatoren und insbesondere als Unkrautbekämpfungsmittel verwendet werden. Überraschenderweise zeigen die erfindungsgemäßen Verbindungen eine breite Wirkung gegen monokotyle und dikotyle Unkräuter bei guter Verträglichkeit gegenüber Kulturpflanzen. Ob die erfindungsgemäßen Verbindungen als totale oder selektive Herbizide wirken, hängt im wesentlichen von der Aufwandmenge, aber auch von der Anwendungsart und dem Anwendungszeitpunkt ab.

Die Anwendung kann im Vorsaat-, Vorauflauf- oder Nachauflaufverfahren erfolgen.

Die erfindungsgemäßen Wirkstoffe können zum Beispiel gegen die folgenden Pflanzengattungen eingesetzt werden:

Dikotyle Unkräuter der Gattungen Polygonum, Sinapis, Atriplex, Spergula, Stellaria, Galium, Viola, Cirsium, Amaranthus, Ipomoea, Xanthium, Abutilon, Chenopodium, Cassia, Convolvulus, Mentha, Veronica, Matricaria, Solanum, Lamium, Thlaspi, Capsella, Datura, Galinsoga, Mercurialis, Rhaphanus, Vicia, Portulaca, Physalis, Sida, Anoda, Euphorbia, Myosotis, Centaurea, Brassica, Chrysanthemum und Helianthus.

Monokotyle Unkräuter der Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Agrostis, Alopecurus, Apera, Rottboellia, Triticum und Hordeum.

Die Verbindungen können in vielen landwirtschaftlichen Hauptkulturen, wie zum Beispiel Weizen, Gerste, Reis und Baumwolle, angewendet werden.

Die Verwendung der erfindungsgemäßen Wirkstoffe ist keineswegs auf die genannten Unkrautgattungen und Kulturpflanzen beschränkt, sondern erstreckt sich in gleicher Weise auf andere Pflanzen.

Die Verbindungen eignen sich auch zur Unkrautbekämpfung auf Industrie- und Gleisanlagen, auf Wegen und Plätzen mit und ohne Baumbewuchs, in Forst-, Ziergehölz-, Beerenfrucht- und Hopfenanlagen sowie in Plantagenkulturen.

Die angewandte Wirkstoffmenge kann in einem größeren Bereich schwanken. Sie hängt im wesentlichen von der Art des gewünschten Effekts ab. Im allgemeinen liegen die Aufwandmengen zwischen 0,01 und 1 kg Wirkstoff pro Hektar Bodenfläche, vorzugsweise zwischen 0,1 und 0,5 kg Wirkstoff pro Hektar.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der

Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

**A) Spritzpulver**

1.) 25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins

2.) 40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

**B) Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

**C) Emulsionskonzentrat**

25 Gewichtsprozent Wirkstoff
15 Gewichtsprozent Cyclohexanon
55 Gewichtsprozent Xylol
5 Gewichtsprozent einer Mischung aus dem Calciumsalz der Dodecylbenzol sulfonsäure und Nonylphenylpolyoxyethylen

Die nachstehenden Beispiele beschreiben die Herstellung der erfindungsgemäßen Verbindungen:

**Beispiel 1**

3-Methoxycarbonyl-5,6-dimethyl-7-oxo-6,7-dihydro-pyrazolo[1,5-a][1,3,5]triazin-2-sulfonsäure-2,6-dichloranilid (nach Verfahrensvariante A))

2,84 g (17,5 mmol) 2,6-Dichloranilin werden in 15 ml Pyridin mit 5,00 g (15,6 mmol) 3-Methoxycarbonyl-5,6-dimethyl-7-oxo-6,7-dihydro-pyrazolo[1,5-a][1,3,5]triazin-2-sulfonsäurechlorid 15 Stunden bei 50 °C gerührt und die unlöslichen Rückstände abfiltriert. Das Filtrat wird eingeengt, der Rückstand mit 2 N Salzsäure versetzt, das Produkt abgesaugt, mit Hexan/Essigester-Gemisch über Kieselgel chromatographiert und aus Essigester umkristallisiert.
Ausbeute: 1,97 g = 28 % der Theorie
Fp.: 265-267 °C

Elementaranalyse

ber. (%):
C 40,37 H 2,94 N 15,69 S 7,19 Cl 15,89
gef. (%):
C 40,45 H 3,34 N 14,94 S 7,21 Cl 15,70

**Herstellung der Ausgangsverbindungen zu Beispiel 1**

**a)** 3-Methoxycarbonyl-5,6-dimethyl-7-oxo-6,7-dihydro-pyrazolo[1,5-a][1,3,5]triazin-2-sulfonsäurechlorid

15,50 g (43 mmol) 2-Benzylthio-3-methoxycarbonyl-5,6-dimethyl-6,7-dihydropyrazolo[1,5-a][1,3,5]triazin-7-thion werden in 200 ml Wasser/Eisessig (1:1-Gemisch) suspendiert. Bei 10 °C wird 3 Stunden Chlor eingeleitet. Das Produkt wird mit Methylenchlorid extrahiert und über Magnesiumsulfat getrocknet. Nach dem Einengen wird der Rückstand mit Ether versetzt und das Produkt abgesaugt.
Ausbeute: 9,70 = 70 % der Theorie
Fp.: 188-190 °C

**b)** 2-Benzylthio-3-methoxycarbonyl-5,6-dimethyl-6,7-dihydro-pyrazolo[1,5-a][1,3,5]triazin-7-thion

6,77 g (20,1 mmol) 5-Amino-3-benzylthio-4-methoxycarbonyl-pyrazol-1-thiocarbonsäure-methylamid werden in 35 ml Orthoessigsäuretriethylester 20 Stunden bei 135 °C gerührt, wobei das gebildete Ethanol abdestilliert wird. Nach Abkühlen auf 5 °C wird das Produkt abgesaugt, mit Ether gewaschen und getrocknet.
Ausbeute: 5,35 g = 74 % der Theorie
Fp.: 212-213 °C

Elementaranalyse

ber. (%):
C 53,31 H 4,47 N 15,54 S 17,79
gef. (%):
C 53,37 H, 4,27 N 15,56 S 18,11

**c)** 5-Amino-3-benzylthio-4-methoxycarbonyl-pyrazol-1-thiocarbonsäure-methylamid

45,11 g (0,127 mol) 3,3-Dibenzylthio-2-cyano-acrylsäuremethylester und 13,35 g (0,127 mol) 4-Methylthiosemicarbazid werden in 225 ml Ethanol 10 Stunden zum Sieden erhitzt. Nach Abkühlen auf 5 °C wird das Produkt abgesaugt, mit kaltem Ether gewaschen und getrocknet.
Ausbeute: 33,61 g = 79 % der Theorie
Fp.: 162-163 °C

Elementaranalyse

ber. (%):
C 49,98 H 4,79 N 16,65 S 19,06
gef. (%):
C 50,00 H 5,12 N 16,78 S 19,48

**d)** 3,3-Dibenzylthio-2-cyano-acrylsäuremethylester

Aus 80,49 g (3,5 mol) Natrium und 1050 ml Methanol wird eine Natriummethylat-Lösung hergestellt. In 600 ml dieser Lösung werden 200,2 g (2,0 mol) 99 %iger Cyanessigsäuremethylester gelöst, auf 5 °C abgekühlt und 60 ml (1,0 mol) Schwefelkohlenstoff unter Kühlung zugetropft. Es werden weitere 300 ml Methylatlösung hinzugegeben und die Mischung auf 10 °C gekühlt. 30 ml (0,5 mol) Schwefelkohlenstoff werden dazugetropft, danach die restliche Methylatlösung hinzugefügt und schließlich weitere 15,75 ml (0,25 mol) Schwefelkohlenstoff dazugetropft. Es wird 30 Minuten bei 15 °C gerührt. Unter Kühlung werden 598,68 g (3,5 mol) Benzylbromid dazugetropft. Die Mischung wird 2 Stunden bei 20 °C gerührt, das Lösungsmittel abdestilliert und der Rückstand mit Eiswasser versetzt. Das Produkt wird abgesaugt, mit Wasser und Ether gewaschen und getrocknet.
Ausbeute: 521,2 g = 84 % der Theorie
Fp.: 100-101 °C

Elementaranalyse

ber. (%):
c 64,19 H 4,82 N 3,94 S 18,04
gef. (%):
C 64,14 H 4,82 N 4,18 S 17,82

**Beispiel 2**

3-Methoxycarbonyl-5,6-dimethyl-7-thioxo-6,7-dihydro-pyrazolo[1,5-a][1,3,5]triazin-2-sulfonsäure-2,6-difluoranilid (nach Verfahrensvariante C))

2,6 g (6,7 mmol) 4-Methoxycarbonyl-5-(1-methoxy-ethylidenamino)-pyrazol-3-sulfonsäure-2,6-difluoranilid werden mit 20 ml Tetrahydrofuran, 0,88 g (12 mmol) Methylisothiocyanat und 0,71 g (7 mmol) Triethylamin 48 Stunden bei 25 °C und 8 Stunden bei 50 °C gerührt. Die Mischung wird auf 5 °C abgekühlt, die Kristalle abgesaugt und mit Tetrahydrofuran und Ether nachgewaschen.
Ausbeute: 1,5 g = 52 % der Theorie
Fp.: 284-286 °C

Elementaranalyse

ber. (%):
C 41,95 H 3,05 N 16,31 S 14,93 F 8,85
gef. (%):
C 41,89 H 3,49 N 16,23 S 15,09 F 8,91

**Herstellung der Ausgangsverbindungen zu Beispiel 2**

**a)** 4-Methoxycarbonyl-5-(1-methoxy-ethylidenamino)-pyrazol-3-sulfonsäure-2,6-difluoranilid

5,0 g (15 mmol) 5-Amino-4-methoxycarbonyl-pyrazol-3-sulfonsäure-2,6-difluoranilid werden mit 50 ml Acetonitril, 2,1 g (17,5 mmol) Orthoessigsäuretrimethylester und 3 Tropfen Essigsäure 8 Stunden unter Rückfluß erhitzt. Die unlöslichen Anteile werden abfiltriert und das Filtrat eingeengt. Der Rückstand wird mit Hexan/Essigester-Gemisch über Kieselgel chromatographiert und aus Ether kristallisiert.
Ausbeute: 4,8 g = 82 % der Theorie
Fp.: 194-196 °C

Elementaranalyse

ber. (%):
C 43,30 H 3,63 N 14,43 S 8,26 F 9,78
gef. (%):
C 43,27 H 3,98 N 14,13 S 8,09 F 9,69

**b)** 5-Amino-4-methoxycarbonyl-pyrazol-3-sulfonsäure-2,6-difluoranilid

7,50 g (20 mmol) 5-Acetylamino-4-methoxycarbonyl-pyrazol-3-sulfonsäure-2,6-difluoranilid werden in 80 ml 4,4 N methanolischer Salzsäure-Lösung suspendiert und 5 Stunden unter Rückfluß erhitzt. Das Lösungsmittel wird abdestilliert und der Rückstand mit Wasser versetzt. Das Produkt wird abgesaugt, mit Wasser gewaschen und getrocknet.
Ausbeute: 6,2 g = 93 % der Theorie
Fp.: 265-266 °C

Elementaranalyse

ber. (%):
C 39,76 H 3,03 N 16,86 S 9,65 F 11,44
gef. (%):
C 40,23 H 3,25 N 17,12 S 9,80 F 11,46

**c)** 5-Acetylamino-4-methoxycarbonyl-pyrazol-3-sulfonsäure-2,6-difluoranilid

23,63 g (177,5 mmol) 97 %iges 2,6-Difluoranilin und 1,48 g N,N-Dimethyl-4-aminopyridin werden in 255 ml Pyridin gelöst und 25,0 g (88,75 mmol) 5-Acetylamino-4-methoxycarbonyl-pyrazol-3-sulfonsäurechlorid portionsweise hinzugefügt. Die Mischung wird 48 Stunden bei 25° C gerührt. Das Pyridin wird abdestilliert und der Rückstand in 510 ml Methylenchlorid gelöst, zweimal mit 370 ml 5 N Salzsäure-Lösung und zweimal mit 370 ml Wasser gewaschen, über Magnesiumsulfat getrocknet und eingeengt. Der Rückstand wird mit Ether verrieben, das Produkt abgesaugt, mit Ether gewaschen und getrocknet.
Ausbeute: 17,53 % = 53 % der Theorie
Fp.: 221-222° C

Elementaranalyse

ber. (%):
C 41,71 H 3,23 N 14,97 S 8,57 F 10,15
gef. (%):
C 41,66 H 3,30 N 14,83 S 8,54 F 10,23

**d)** 5-Acetylamino-4-methoxycarbonyl-pyrazol-3-sulfonsäurechlorid

73,0 g (239 mmol) 5-Acetylamino-3-benzylthio-pyrazol-4-carbonsäuremethylester werden in 470 ml Methylenchlorid gelöst und mit 124,15 g Kieselgel und 25,6 ml Wasser versetzt und auf 0° C gekühlt. Bei 0 bis 5° C werden 160,6 g (1,19 mol) Sulfurylchlorid in 730 ml Methylenchlorid dazugetropft. Die Mischung wird 3 Stunden bei 0° C geréhrt, filtriert, mehrmals mit Methylenchlorid nachgewaschen, über Magnesiumsulfat getrocknet und eingeengt. Der Rückstand wird mit Ether/Hexan verrieben, die Kristalle abgesaugt, mit Hexan gewaschen und getrocknet.
Ausbeute: 51,9 g = 77 % der Theorie
Fp.: 143-144° C

Elementaranalyse

ber. (%):
c 29,85 H 2,86 N 14,92 S 11,38 Cl 12,59
gef. (%):
C 29,95 H 2,76 N 14,87 S 11,40 Cl 12,51

**e)** 5-Acetylamino-3-benzylthio-pyrazol-4-carbonsäuremethylester

70,5 g (0,27 mol) 5-Amino-3-benzylthio-pyrazol-4-carbonsäuremethylester werden bei 80° C in 135 ml Eisessig gelöst und mit 27,75 g (0,27 mol) Aceetanhydrid versetzt. Die Lösung wird 7 Stunden unter Rückfluß erhitzt und eingeengt. Der Rückstand wird mit Ether kristallisiert, das Produkt abgesaugt, mit Ether

gewaschen und getrocknet.
Ausbeute: 73,2 g = 89 % der Theorie
Fp.: 136-137 °C

Elementaranalyse

ber. (%):
C 55,07 H 4,95 N 13,76 S 10,50
gef. (%):
C 55,17 H 4,77 N 13,80 S 10,79

**f)** 5-Amino-3-benzylthio-pyrazol-4-carbonsäuremethylester

124,34 g (0,35 mol) 3,3-Dibenzylthio-2-cyano-acrylsäuremethylester und 46,70 g (0,35 mol) Hydrazinoameisensäure-tert.-butylester werden in 700 ml Ethanol 3,5 Stunden unter Rückfluß erhitzt und eingeengt. Der Rückstand wird mit 700 ml Eisessig versetzt und 3 Stunden bei 110 °C und 1 Stunde bei 5 °C gerührt. Das Produkt wird abgesaugt, mit Eisessig und Hexan gewaschen und getrocknet.
Ausbeute: 70,9 g = 77 % der Theorie
Fp.: 111-112 °C

Elementaranalyse

ber. (%):
C 54,73 H 4,97 N 15,96
gef. (%):
C 54,02 H 4,68 N 15,93

**Beispiel 3**

3-Methoxycarbonyl-5,6-dimethyl-7-oxo-6,7-dihydro-pyrazolo[1,5-a][1,3,5]triazin-2-sulfonsäure-2,6-difluoranilid (nach Verfahrensvariante C))

1,5 g (3,9 mmol) 4-Methoxycarbonyl-5-(1-methoxyethylidenamino)-pyrazol-3-sulfonsäure-2,6-difluoranilid werden mit 20 ml Tetrahydrofuran, 0,29 g (5,0 mmol) Methylisocyanat und 0,39 g (3,9 mmol) Triethylamin 48 Stunden bei 25 °C gerührt. Die Kristalle werden dann abgesaugt und mit Tetrahydrofuran und Ether nachgewaschen.
Ausbeute: 1,3 g = 81 % der Theorie
Fp.: 257-259 °C

Elementaranalyse

ber. (%):
C 43,58 H 3,17 N 16,94 S 7,76 F 9,19
gef. (%):
C 43,34 H 3,23 N 16,80 S 7,53 F 9,03

Analog zu den Beispielen 1 bis 3 lassen sich folgende erfindungsgemäße Verbindungen herstellen:

| Beispiel Nr. | Name der Verbindung | Physikalische Konstante |
|---|---|---|
| 4 | 3-Methoxycarbonyl-5,6-dimethyl-7-thioxo-6,7-dihydro-pyrazolo[1,5-a]-[1,3,5]triazin-2-sulfonsäure-2,6-dichloranilid | Fp.: 304-305 °C |
| 5 | 3-Methoxycarbonyl-5,6-dimethyl-7-oxo-6,7-dihydro-pyrazolo[1,5-a][1,3,5]-triazin-2-sulfonsäure-2,6-dichlor-3-methylanilid | Fp.: 262-264 °C |
| 6 | 3-Methoxycarbonyl-5,6-dimethyl-7-thioxo-6,7-dihydro-pyrazolo[1,5-a]-[1,3,5]triazin-2-sulfonsäure-2,6-dichlor-3-methylanilid | Fp.: 312-314 °C |
| 7 | 3-Methoxycarbonyl-5,6-dimethyl-7-oxo-6,7-dihydro-pyrazolo[1,5-a][1,3,5]triazin-2-sulfonsäure-2-chlor-6-fluoranilid | Fp.: 240-242 °C |
| 8 | 3-Methoxycarbonyl-5,6-dimethyl-7-thioxo-6,7-dihydro-pyrazolo[1,5-a][1,3,5]triazin-2-sulfonsäure-2-chlor-6-fluoranilid | Fp.: 285-286 °C |
| 9 | 3-Methoxycarbonyl-5,6-dimethyl-7-oxo-6,7-dihydro-pyrazolo[1,5-a][1,3,5]triazin-2-sulfonsäure-2-chlor-6-methylanilid | Fp.: 259-261 °C |
| 10 | 3-Methoxycarbonyl-5,6-dimethyl-7-thioxo-6,7-dihydro-pyrazolo[1,5-a][1,3,5]triazin-2-sulfonsäure-2-chlor-6-methylanilid | Fp.: 295-297 °C |

| Beispiel Nr. | Name der Verbindung | Physikalische Konstante |
|---|---|---|
| 11 | 3-Methoxycarbonyl-5,6-dimethyl-7-oxo-6,7-dihydro-pyrazolo[1,5-a][1,3,5]triazin-2-sulfonsäure-2,6-dibromanilid | Fp.: 293-295 °C |
| 12 | 3-Methoxycarbonyl-5,6-dimethyl-7-thioxo-6,7-dihydro-pyrazolo[1,5-a][1,3,5]triazin-2-sulfonsäure-2,6-dibromanilid | Fp.: 327-330 °C |
| 13 | 3-Methoxycarbonyl-5,6-dimethyl-7-oxo-6,7-dihydro-pyrazolo[1,5-a][1,3,5]triazin-2-sulfonsäure-2-methyl-6-nitroanilid-Hydrat | Fp.: 230-232 °C |
| 14 | 3-Methoxycarbonyl-5,6-dimethyl-7-thioxo-6,7-dihydro-pyrazolo[1,5-a][1,3,5]triazin-2-sulfonsäure-2-methyl-6-nitroanlid | Fp.: 271-274 °C |
| 15 | 3-Methoxycarbonyl-5,6-dimethyl-7-thioxo-6,7-dihydro-pyrazolo[1,5-a][1,3,5]triazin-2-sulfonsäure-2-metoxycarbonyl-6-methylanilid | Fp.: 210-212 °C |
| 16 | 3-Methoxycarbonyl-5,6-dimethyl-7-thioxo-6,7-dihydro-pyrazolo[1,5-a][1,3,5]triazin-2-sulfonsäure-2-brom-6-methoxyanilid x Hydrat | Fp.: 276-278 °C |
| 17 | 3-Ethoxycarbonyl-5,6-dimethyl-7-oxo-6,7-dihydro-pyrazolo[1,5-a][1,3,5]triazin-2-sulfonsäure-2,6-dichloranilid | Fp.: 283-284 °C |
| 18 | 3-Ethoxycarbonyl-5,6-dimethyl-7-oxo-6,7-dihydro-pyrazolo[1,5-a][1,3,5]triazin-2-sulfonsäure-2,6-difluoranilid | Fp.: 221-223 °C |

| Beispiel Nr. | Name der Verbindung | Physikalische Konstante |
|---|---|---|
| 19 | 3-Ethoxycarbonyl-5,6-dimethyl-7-oxo-6,7-dihydro-pyrazolo[1,5-a][1,3,5]triazin-2-sulfonsäure-2-chlor-6-fluoranilid | Fp.: 214-217 ° C |
| 20 | 3-Ethoxycarbonyl-5,6-dimethyl-7-oxo-6,7-dihydro-pyrazolo[1,5-a][1,3,5]triazin-2-sulfonsäure-2,6-dichlor-3-methylanilid | Fp.: 228-230 ° C |
| 21 | 3-Ethoxycarbonyl-5,6-dimethyl-7-oxo-6,7-dihydro-pyrazolo[1,5-a][1,3,5]triazin-2-sulfonsäure-2,6-dibromanilid | Fp.: 295-297 ° C |
| 22 | 3-Ethoxycarbonyl-5,6-dimethyl-7-oxo-6,7-dihydro-pyrazolo[1,5-a][1,3,5]triazin-2-sulfonsäure-2-brom-6-chloranilid | Fp.: 285-286 ° C |
| 23 | 3-Ethoxycarbonyl-5,6-dimethyl-7-thioxo-6,7-dihydro-pyrazolo[1,5-a][1,3,5]triazin-2-sulfonsäure-2,6-dichloranlid | Fp.: 272-274 ° C |
| 24 | 3-Ethoxycarbonyl-5,6-dimethyl-7-thioxo-6,7-dihydro-pyrazolo[1,5-a][1,3,5]triazin-2-sulfonsäure-2-methoxycarbonyl-6-methylanilid | Fp.: 182-186 ° C |
| 25 | 3-Ethoxycarbonyl-5,6-dimethyl-7-thioxo-6,7-dihydro-pyrazolo[1,5-a][1,3,5]triazin-2-sulfonsäure-2,6-dichlor-3-methylanilid | Fp.: 257-259 ° C |
| 26 | 3-Ethoxycarbonyl-5,6-dimethyl-7-thioxo-6,7-dihydro-pyrazolo[1,5-a][1,3,5]triazin-2-sulfonsäure-2-chlor-6-fluoranilid | Fp.: 232-235 ° C |

| Beispiel Nr. | Name der Verbindung | Physikalische Konstante |
|---|---|---|
| 27 | 3-Ethoxycarbonyl-5,6-dimethyl-7-thioxo-6,7-dihydro-pyrazolo[1,5-a][1,3,5]triazin-2-sulfonsäure-2,6-difluoranilid | Fp.: 214-216 °C |
| 28 | 3-Ethoxycarbonyl-5,6-dimethyl-7-thioxo-6,7-dihydro-pyrazolo[1,5-a][1,3,5]triazin-2-sulfonsäure-2-chlor-6-methylanilid | Fp.: 225-226 °C |
| 29 | 3-Ethoxycarbonyl-5,6-dimethyl-7-thioxo-6,7-dihydro-pyrazolo[1,5-a][1,3,5]triazin-2-sulfonsäure-2,6-dibromanilid | Fp.: 285-287 °C |
| 30 | 3-Ethoxycarbonyl-5,6-dimethyl-7-thioxo-6,7-dihydro-pyrazolo[1,5-a][1,3,5]triazin-2-sulfonsäure-2-brom-6-chloranilid | Fp.: 291-293 °C |
| 31 | 3-Cyano-5,6-dimethyl-7-oxo-6,7-dihydro-pyrazolo[1,5-a][1,3,5]triazin-2-sulfonsäure-2,6-dichloranilid | Fp.: 338-341 °C |
| 32 | 3-Cyano-5,6-dimethyl-7-oxo-6,7-dihydro-pyrazolo[1,5-a][1,3,5]triazin-2-sulfonsäure-2,6-dibromanilid | Fp.: 303 °C (Zers.) |
| 33 | 3-Cyano-5,6-dimethyl-7-oxo-6,7-dihydro-pyrazolo[1,5-a][1,3,5]triazin-2-sulfonsäure-2,6-dichlor-3-methylanilid | Fp.: 298-303 °C |
| 34 | 3-Cyano-5,6-dimethyl-7-oxo-6,7-dihydro-pyrazolo[1,5-a][1,3,5]triazin-2-sulfonsäure-2-methoxycarbonyl-6-methylanilid | Fp.: 258-262 °C |

| Beispiel Nr. | Name der Verbindung | Physikalische Konstante |
|---|---|---|
| 35 | 3-Cyano-5,6-dimethyl-7-thioxo-6,7-dihydro-pyrazolo[1,5-a][1,3,5]triazin-2-sulfonsäure-2,6-dichloranilid | Fp.: 279-282 °C |
| 36 | 3-Cyano-5,6-dimethyl-7-thioxo-6,7-dihydro-pyrazolo[1,5-a][1,3,5]triazin-2-sulfonsäure-2,6-dichlor-3-methylanilid | Fp.: 272-273 °C |
| 37 | 3-Cyano-5,6-dimethyl-7-thioxo-6,7-dihydro-pyrazolo[1,5-a][1,3,5]triazin-2-sulfonsäure-2,6-dibromanilid | Fp.: 300 °C (Zers.) |
| 38 | 3-Cyano-5,6-dimethyl-7-thioxo-6,7-dihydro-pyrazolo[1,5-a][1,3,5]triazin-2-sulfonsäure-2-methoxycarbonyl-6-methylanilid | Fp.: 248-249 °C |
| 39 | 3-Aminocarbonyl-5,6-dimethyl-7-oxo-6,7-dihydro-pyrazolo[1,5-a][1,3,5]triazin-2-sulfonsäure-2,6-dichloranilid | Fp.: 303-305 °C |
| 40 | 3-Aminocarbonyl-5,6-dimethyl-7-oxo-6,7-dihydro-pyrazolo[1,5-a][1,3,5]triazin-2-sulfonsäure-2,6-dichlor-3-methylanilid | Fp.: 307 °C (Zers.) |
| 41 | 3-Aminocarbonyl-5,6-dimethyl-7-oxo-6,7-dihydro-pyrazolo[1,5-a][1,3,5]triazin-2-sulfonsäure-2,6-dibromanilid | Fp.: 274-275 °C |
| 42 | 3-Aminocarbonyl-5,6-dimethyl-7-thioxo-6,7-dihydro-pyrazolo[1,5-a][1,3,5]triazin-2-sulfonsäure-2,6-dichloranilid | Fp.: 295-296 °C |

| Beispiel Nr. | Name der Verbindung | Physikalische Konstante |
|---|---|---|
| 43 | 3-Aminocarbonyl-5,6-dimethyl-7-thioxo-6,7-dihydro-pyrazolo[1,5-a][1,3,5]triazin-2-sulfonsäure-2,6-dichlor-3-methylanilid | Fp.: 269 °C (Zers.) |
| 44 | 3-Aminocarbonyl-5,6-dimethyl-7-thioxo-6,7-dihydro-pyrazolo[1,5-a][1,3,5]triazin-2-sulfonsäure-2,6-dibromanilid | Fp.: 279-280 °C |
| 45 | 3-Methylsulfonyl-5,6-dimethyl-7-oxo-6,7-dihydro-pyrazolo[1,5-a][1,3,5]triazin-2-sulfonsäure-2,6-dichloranilid | Fp.: 289-292 °C |
| 46 | 3-Methylsulfonyl-5,6-dimethyl-7-oxo-6,7-dihydro-pyrazolo[1,5-a][1,3,5]triazin-2-sulfonsäure-2,6-dichlor-3-methylanilid | Fp.: 273-275 °C |
| 47 | 3-Methylsulfonyl-5,6-dimethyl-7-thioxo-6,7-dihydro-pyrazolo[1,5-a][1,3,5]triazin-2-sulfonsäure-2,6-dichloranilid | Fp.: 318-320 °C |
| 48 | 3-Methylsulfonyl-5,6-dimethyl-7-thioxo-6,7-dihydro-pyrazolo[1,5-a][1,3,5]triazin-2-sulfonsäure-2,6-dichlor-3-methylanilid | Fp. 290-293 °C |
| 49 | 3-Methoxycarbonyl-5,6-dimethyl-7-oxo-6,7-dihydro-pyrazolo[1,5-a][1,3,5]triazin-2-sulfonsäure-2-methoxycarbonyl-6-methylanilid | |
| 50 | 3-Methoxycarbonyl-5,6-dimethyl-7-oxo-6,7-dihydro-pyrazolo[1,5-a][1,3,5]triazin-2-sulfonsäure-2-brom-6-methoxyanilid | |

| Beispiel Nr. | Name der Verbindung | Physikalische Konstante |
|---|---|---|
| 51 | 3-Methoxycarbonyl-5,6-dimethyl-7-oxo-6,7-dihydro-pyrazolo[1,5-a][1,3,5]triazin-2-sulfonsäure-2-trifluormethylanilid | |
| 52 | 3-Methoxycarbonyl-5,6-dimethyl-7-oxo-6,7-dihydro-pyrazolo[1,5-a][1,3,5]triazin-2-sulfonsäure-2-nitro-5,6-dimethylanilid | |
| 53 | 3-Methoxycarbonyl-5,6-dimethyl-7-oxo-6,7-dihydro-pyrazolo[1,5-a][1,3,5]triazin-2-sulfonsäure-2-brom-6-chloranilid | |
| 54 | 3-Methoxycarbonyl-5,6-dimethyl-7-oxo-6,7-dihydro-pyrazolo[1,5-a][1,3,5]triazin-2-sulfonsäure-2-brom-6-chlor-3-methylanilid | |
| 55 | 3-Methoxycarbonyl-5,6-dimethyl-7-oxo-6,7-dihydro-pyrazolo[1,5-a][1,3,5]triazin-2-sulfonsäure-2-brom-6-chlor-5-methylanilid | |
| 56 | 3-Methoxycarbonyl-5,6-dimethyl-7-oxo-6,7-dihydro-pyrazolo[1,5-a][1,3,5]triazin-2-sulfonsäure-2,6-dibrom-3-methylanilid | |
| 57 | 3-Methoxycarbonyl-5,6-dimethyl-7-oxo-6,7-dihydro-pyrazolo[1,5-a][1,3,5]triazin-2-sulfonsäure-2-trifluormethyl-6-methylanilid | |
| 58 | 3-Methoxycarbonyl-5,6-dimethyl-7-oxo-6,7-dihydro-pyrazolo[1,5-a][1,3,5]triazin-2-sulfonsäure-2-difluormethoxy-6-methylanilid | |

| Beispiel Nr. | Name der Verbindung | Physikalische Konstante |
|---|---|---|
| 59 | 3-Methoxycarbonyl-5,6-dimethyl-7-oxo-6,7-dihydro-pyrazolo[1,5-a][1,3,5]triazin-2-sulfonsäure-3-6-dimethyl-2-nitroanilid | |
| 60 | 3-Methoxycarbonyl-5,6-dimethyl-7-oxo-6,7-dihydro-pyrazolo[1,5-a][1,3,5]triazin-2-sulfonsäure-2,6-bis-difluormethoxyanilid | |
| 61 | 3-Methoxycarbonyl-5,6-dimethyl-7-oxo-6,7-dihydro-pyrazolo[1,5-a][1,3,5]triazin-2-sulfonsäure-6-fluor-2-methoxycarbonyl-3-methylanilid | |
| 62 | 3-Methoxycarbonyl-5,6-dimethyl-7-oxo-6,7-dihydro-pyrazolo[1,5-a][1,3,5]triazin-2-sulfonsäure-2-methoxy-6-trifluormethylanilid | |
| 63 | 3-Methoxycarbonyl-5,6-dimethyl-7-thioxo-6,7-dihydro-pyrazolo[1,5-a][1,3,5]triazin-2-sulfonsäure-2-trifluormethylanilid | |
| 64 | 3-Methoxycarbonyl-5,6-dimethyl-7-thioxo-6,7-dihydro-pyrazolo[1,5-a][1,3,5]triazin-2-sulfonsäure-2-nitro-5,6-dimethylanilid | |
| 65 | 3-Methoxycarbonyl-5,6-dimethyl-7-thioxo-6,7-dihydro-pyrazolo[1,5-a][1,3,5]triazin-2-sulfonsäure-2-brom-6-chloranilid | |
| 66 | 3-Methoxycarbonyl-5,6-dimethyl-7-thioxo-6,7-dihydro-pyrazolo[1,5-a][1,3,5]triazin-2-sulfonsäure-2-brom-6-chlor-3-methylanilid | |

| Beispiel Nr. | Name der Verbindung | Physikalische Konstante |
|---|---|---|
| 67 | 3-Methoxycarbonyl-5,6-dimethyl-7-thioxo-6,7-dihydro-pyrazolo[1,5-a][1,3,5]triazin-2-sulfonsäure-2-brom-6-chlor-5-methylanilid | |
| 68 | 3-Methoxycarbonyl-5,6-dimethyl-7-thioxo-6,7-dihydro-pyrazolo[1,5-a][1,3,5]triazin-2-sulfonsäure-2,6-dibrom-3-methylanilid | |
| 69 | 3-Methoxycarbonyl-5,6-dimethyl-7-thioxo-6,7-dihydro-pyrazolo[1,5-a][1,3,5]triazin-2-sulfonsäure-2-trifluormethyl-6-methylanilid | |
| 70 | 3-Methoxycarbonyl-5,6-dimethyl-7-thioxo-6,7-dihydro-pyrazolo[1,5-a][1,3,5]triazin-2-sulfonsäure-2-difluormethoxy-6-methylanilid | |
| 71 | 3-Methoxycarbonyl-5,6-dimethyl-7-thioxo-6,7-dihydro-pyrazolo[1,5-a][1,3,5]triazin-2-sulfonsäure-3,6-dimethyl-2-nitroanilid | |
| 72 | 3-Methoxycarbonyl-5,6-dimethyl-7-thioxo-6,7-dihydro-pyrazolo[1,5-a][1,3,5]triazin-2-sulfonsäure-2,6-bis-difluormethoxyanilid | |
| 73 | 3-Methoxycarbonyl-5,6-dimethyl-7-thioxo-6,7-dihydro-pyrazolo[1,5-a][1,3,5]triazin-2-sulfonsäure-6-fluor-2-methoxycarbonyl-3-methylanilid | |

| Beispiel Nr. | Name der Verbindung | Physikalische Konstante |
|---|---|---|
| 74 | 3-Methoxycarbonyl-5,6-dimethyl-7-thioxo-6,7-dihydro-pyrazolo[1,5-a][1,3,5]triazin-2-sulfonsäure-2-methoxy-6-trifluormethylanilid | |
| 75 | 3-Ethoxycarbonyl-5,6-dimethyl-7-thioxo-6,7-dihydro-pyrazolo[1,5-a][1,3,5]triazin-2-sulfonsäure-2-brom-6-methoxyanilid | Fp.: 210 °C |
| 76 | 3-Aminocarbonyl-5,6-dimethyl-7-oxo-6,7-dihydro-pyrazolo[1,5-a][1,3,5]triazin-2-sulfonsäure-2-carboxy-6-methylanilid | Fp.: 257-258 °C |
| 77 | 3-Aminocarbonyl-5,6-dimethyl-7-thioxo-6,7-dihydro-pyrazolo[1,5-a][1,3,5]triazin-2-sulfonsäure-2-carboxy-6-methylanilid | Fp.: 267-268 °C |

Die nachfolgenden Beispiele erläutern die Anwendungsmöglichkeiten der erfindungsgemäßen Verbindungen:

Beispiel A

Samen mono- und dikotyler Pflanzenarten wurden in Schalen mit humushaltigem Sandboden ausgelegt und mit Erde abgedeckt. Die erfindungsgemäßen Verbindungen wurden als Suspensionen mit 500 Liter Wasser/ha in einer Aufwandmenge von 0,3 kg Wirkstoff/ha vor dem Auflaufen der Pflanzen auf die Erdoberfläche appliziert.

Nach der Behandlung wurden die Versuchstöpfe im Gewäcnshaus aufgestellt und die Versuchspflanzen unter guten Wuchsbedingungen kultiviert. Drei Wochen nach der Behandlung wurden die Pflanzenschäden bonitiert. Als Vergleich dienten dabei unbehandelte Kontrollen.

Wie aus der Tabelle ersichtlich wird, wurden zahlreiche Pflanzenarten vernichtet beziehungsweise geschädigt. Weizen und Gerste waren widerstandsfähig.

In der folgenden Tabelle bedeuten:

0 = keine Wirkung - = nicht geprüft

4 = Vernichtung der Pflanzen

Tr = Triticum aestivum

Ho = Hordeum distichum

Br = Brassica napus napus

He = Helianthus annuus

St = Stellaria media

Ab = Abutilon hybridum

Ma = Matricaria chamomilla

Vi = Viola tricolor

Ch = Chrysanthemum segetum

So = Sorghum sativum

Ec = Echinochloa crus-galli

Se = Setaria italica

Ga = Galium aparine

| Erfindungsgemäße Verbindung | Tr | Ho | Br | He | St | Ab | Ma | Vi | Ch | So | Ec | Se | Ga |
|---|---|---|---|---|---|---|---|---|---|---|---|---|---|
| Beispiel 1 | 0 | 0 | 3 | 3 | 2 | 3 | 4 | 4 | 4 | 3 | 3 | 3 | 3 |
| Beispiel 2 | 2 | 2 | 3 | 3 | 2 | 2 | 4 | 3 | 3 | 4 | 4 | 3 | 3 |
| Beispiel 3 | 0 | 0 | 3 | 3 | 1 | 2 | 4 | 3 | 3 | 3 | 3 | 4 | 2 |
| Beispiel 4 | 1 | 0 | 3 | 3 | 3 | 3 | 4 | 3 | 3 | 3 | 3 | 4 | 4 |
| Beispiel 5 | 0 | 0 | 3 | 3 | 3 | 3 | 4 | 3 | 4 | 2 | 2 | 2 | 3 |
| Beispiel 6 | 0 | 1 | 2 | 3 | 3 | 2 | 4 | 4 | 4 | 3 | 3 | 2 | 4 |
| Beispiel 7 | 0 | 1 | 2 | 3 | 2 | 2 | 4 | 3 | 3 | 2 | 2 | 2 | 2 |
| Beispiel 8 | 1 | 2 | 3 | 3 | 2 | 2 | 4 | 3 | 4 | 4 | 3 | 4 | 3 |
| Beispiel 9 | 0 | 1 | 2 | 3 | 2 | 2 | 4 | 3 | 3 | 3 | 2 | 3 | 3 |
| Beispiel 10 | 1 | 1 | 3 | 3 | 3 | 2 | 4 | 3 | 3 | 3 | 3 | 2 | 3 |
| Beispiel 15 | 0 | - | 1 | 1 | - | 0 | 4 | 0 | - | - | - | 0 | 0 |
| Beispiel 16 | 1 | - | 1 | 2 | - | 0 | 4 | - | - | - | - | 1 | 2 |
| Beispiel 17 | 0 | - | 3 | 3 | - | 3 | 4 | - | - | - | - | 3 | 3 |
| Beispiel 18 | 0 | - | 3 | 3 | - | 3 | 3 | - | - | - | - | 2 | 2 |
| Beispiel 19 | 0 | - | 3 | 3 | - | 3 | 4 | - | - | - | - | 2 | 2 |
| Beispiel 20 | 0 | - | 3 | 2 | - | 1 | 4 | - | - | - | - | 0 | 2 |
| Beispiel 21 | 1 | - | 3 | 2 | - | 1 | 4 | - | - | - | - | 3 | 3 |
| Beispiel 22 | 0 | - | - | - | - | 3 | 4 | - | - | - | - | 4 | 4 |
| Beispiel 23 | 0 | - | 3 | 3 | - | 3 | 4 | - | - | - | - | 3 | 3 |
| Beispiel 24 | 0 | - | 0 | 0 | - | 0 | 3 | 1 | - | - | - | 0 | 0 |
| Beispiel 25 | 0 | - | 3 | 3 | - | 2 | 4 | - | - | - | - | 2 | 4 |
| Beispiel 26 | 0 | - | 3 | 2 | - | 2 | 4 | - | - | - | - | 0 | 2 |
| Beispiel 27 | 0 | - | 2 | 2 | - | 1 | 4 | - | - | - | - | 0 | 2 |
| Beispiel 28 | 0 | - | 2 | 1 | - | 0 | 3 | - | - | - | - | 0 | 3 |
| Beispiel 29 | 1 | - | 3 | 2 | - | 0 | 4 | - | - | - | - | 0 | 3 |
| Beispiel 30 | 0 | - | - | - | - | 3 | 4 | - | - | - | - | 4 | 4 |
| Beispiel 39 | 0 | - | 3 | 3 | - | 0 | 4 | - | - | - | - | 3 | 3 |
| Beispiel 42 | 1 | - | 3 | 3 | - | 0 | 4 | - | - | - | - | 3 | 3 |
| Beispiel 45 | 0 | - | - | - | - | 0 | 2 | - | - | - | - | 1 | 2 |
| Beispiel 46 | 0 | - | - | - | - | 2 | 4 | - | - | - | - | 2 | 3 |
| Beispiel 47 | 0 | - | - | - | - | 0 | 3 | - | - | - | - | 0 | 2 |
| Beispiel 48 | 0 | - | - | - | - | 1 | 4 | - | - | - | - | 2 | 4 |
| Kontrolle | 0 | 0 | 0 | 0 | 0 | 0 | 0 | 0 | 0 | 0 | 0 | 0 | 0 |

Beispiel B

Samen mono- und dikotyler Pflanzenarten wurden in Schalen mit humushaltigem Sandboden ausgelegt und mit Erde abgedeckt. Die erfindungsgemäßen Verbindungen wurden als Suspensionen mit 500 Liter Wasser/ha in einer Aufwandmenge von 0,3 kg Wirkstoff/ha nach dem Auflaufen der Pflanzen appliziert.

Nach der Behandlung wurden die Versuchstöpfe im Gewächshaus aufgestellt und die Versuchspflanzen unter guten Wuchsbedingungen kultiviert. Zwei Wochen nach der Behandlung wurden die Pflanzenschäden bonitiert. Als Vergleich dienten dabei unbehandelte Kontrollen.

Wie aus der Tabelle ersichtlich wird, wurden zahlreiche Pflanzenarten vernichtet beziehungsweise geschädigt. Weizen und Gerste waren widerstandsfähig.

In der folgenden Tabelle bedeuten:

0 = keine Wirkung
4 = Vernichtung der Pflanzen
Tr = Triticum aestivum
Ho = Hordeum distichum
Br = Brassica napus napus
Ly = Lycopersicon esculentum
Me = Medicago sativa
He = Helianthus annuus
St = Stellaria media
Ab = Abutilon hybridum
Ma = Matricaria chamomilla
Vi = Viola tricolor
Ch = Chrysanthemum segetum
Ga = Galium aparine

| Erfindungsgemäße Verbindung | Tr | Ho | Br | Ly | Me | He | St | Ab | Ma | Vi | Ch | Ga |
|---|---|---|---|---|---|---|---|---|---|---|---|---|
| Beispiel 1 | 0 | 0 | 3 | 4 | 3 | 3 | 4 | 2 | 3 | 2 | 3 | 4 |
| Beispiel 2 | 1 | 0 | 3 | 3 | 3 | 3 | 4 | 2 | 3 | 3 | 3 | 3 |
| Beispiel 3 | 0 | 0 | 3 | 3 | 4 | 3 | 3 | 2 | 2 | 3 | 2 | 3 |
| Beispiel 4 | 1 | 2 | 4 | 4 | 3 | 4 | 4 | 2 | 3 | 1 | 4 | 4 |
| Beispiel 5 | 0 | 0 | 2 | 3 | 3 | 3 | 2 | 2 | 3 | 3 | 4 | 3 |
| Beispiel 6 | 0 | 0 | 3 | 4 | 3 | 4 | 3 | 2 | 4 | 2 | 4 | 4 |
| Beispiel 7 | 0 | 0 | 3 | 3 | 3 | 3 | 2 | 3 | 2 | 2 | 2 | 3 |
| Beispiel 8 | 0 | 2 | 3 | 3 | 3 | 3 | 4 | 3 | 3 | 2 | 4 | 4 |
| Beispiel 9 | 0 | 0 | 2 | 2 | 2 | 2 | 3 | 1 | 2 | 1 | 2 | 3 |
| Beispiel 10 | 0 | 0 | 2 | 3 | 2 | 3 | 3 | 2 | 2 | 2 | 3 | 3 |
| Kontrolle | 0 | 0 | 0 | 0 | 0 | 0 | 0 | 0 | 0 | 0 | 0 | 0 |

## Ansprüche

1. 6,7-Dihydro-pyrazolo[1,5-a][1,3,5]triazin-2-sulfonsäureamide der allgemeinen Formel I

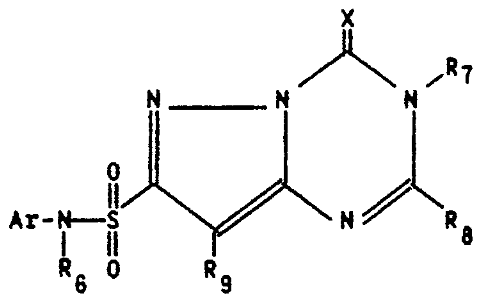

(I) ,

in der
Ar eine Phenyl-, Naphthyl-, Pyridyl- oder Thienylgruppe der allgemeinen Formeln

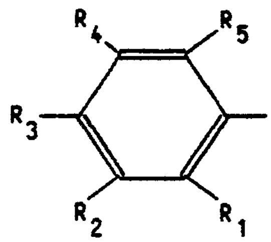 , 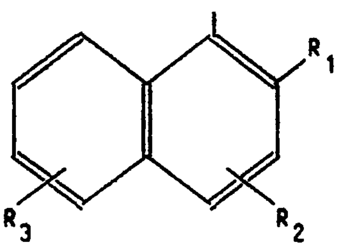 , 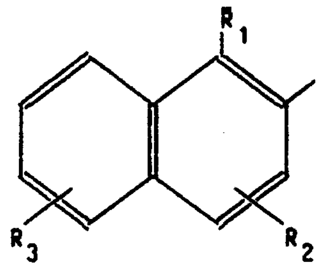 ,

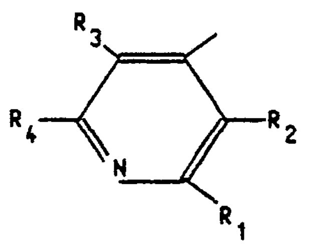 , 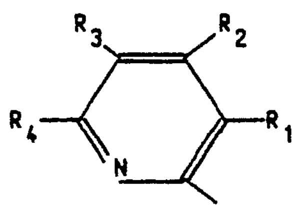 , 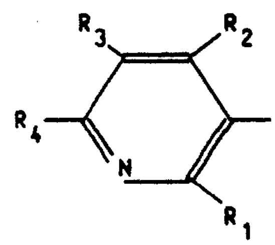 ,

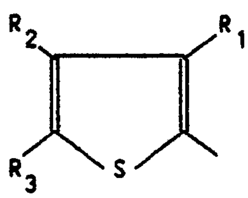 und 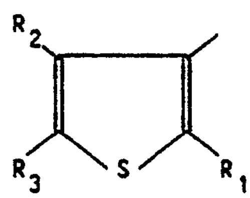 ,

$R_1$, $R_2$, $R_3$, $R_4$ und $R_5$, die gleich oder verschieden sind, ein Wasserstoff atom, einen gegebenenfalls ein- oder mehrfach, gleich oder verschieden durch Halogen, Hydroxy, Halogen-$C_1$-$C_4$-alkyl, $C_1$-$C_4$-Alkoxy, Halogen-$C_1$-$C_4$-alkoxy, $C_1$-$C_4$-Alkylthio, $C_1$-$C_4$-Alkylsulfinyl oder $C_1$-$C_4$-Alkylsulfonyl substitutierten $C_1$-$C_6$-Alkyl-, $C_2$-$C_6$-Alkenyl- oder $C_2$- $C_6$-Alkinylrest, ein Halogenatom, eine $C_1$-$C_4$-Alkoxygruppe, eine Gruppe $R_{10}$-O-CO-, eine Aminocarbonylgruppe $R_{11}R_{12}$N-CO-, eine Aminogruppe $R_{11}R_{12}$N-, eine Cyanogruppe, eine Nitrogruppe, eine Schwefel enthaltende Gruppe $R_{10}$-S(O)$_n$-, eine Acylgruppe $R_{10}$-CO-, eine Gruppe $R_{10}$-O-CO-$(CH_2)_n$-oder einen durch $C_1$-$C_4$-Alkyl, Halogen oder Nitro substituierten Phenyl-oder Phenoxyrest,
$R_6$ ein Wasserstoffatom, eine Acylgruppe $R_{10}$-CO-, eine Gruppe $R_{10}$-O-CO-, eine $C_1$-$C_6$-Alkylgruppe, eine $C_2$-$C_6$-Alkenylgruppe, eine $C_2$-$C_6$-Alkinylgruppe, eine Phenyl-$C_1$-$C_4$-alkylgruppe, eine Aminocarbonylgruppe $R_{11}R_{12}$N-CO-, ein Alkalimetallatom, ein einwertiges Metalläquivalent aus der Gruppe der Erdalkalimetalle und weiterer Metalle oder einen gegebenenfalls durch $C_1$-$C_6$-Alkyl substituierten Ammoniumrest,
$R_7$ und $R_8$, die gleich oder verschieden sind, ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl

substituierten Phenyl-$C_1$-$C_6$-alkyl, Phenyl-$C_2$-$C_6$-alkenyl-oder Phenyl-$C_2$-$C_6$-alkinylrest, einen durch $R_1$, $R_2$, $R_3$, $R_4$ und $R_5$ substituierten Phenylrest, eine Acylgruppe $R_{10}$-CO-, eine Gruppe $R_{10}$-O-CO-, eine Gruppe $R_{10}$-O-CO-$(CH_2)_n$-, eine Aminocarbonylgruppe $R_{11}R_{12}$-N-CO-, oder eine Sulfonylgruppe $R_{10}$-$SO_2$-,

$R_9$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl oder Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest, einen durch $R_1$, $R_2$, $R_3$, $R_4$ und $R_5$ substituierten Phenylrest, eine Acylgruppe $R_{10}$-CO-, eine Gruppe $R_{10}$-O-CO-, eine Gruppe $R_{10}$-O-CO-$(CH_2)_n$-, eine Aminocarbonylgruppe $R_{11}R_{12}$N-CO-, eine Sulfonylgruppe $R_{10}$-$SO_2$- oder $R_{10}$-O-$SO_2$-, eine Aminosulfonylgruppe $R_{11}R_{12}$N-$SO_2$-, eine Cyanogruppe oder eine Nitrogruppe,

$R_{10}$ ein Wasserstoffatom, eine gegebenenfalls ein- oder mehrfach, gleich oder verschieden durch Halogen, Hydroxy oder $C_1$-$C_4$-Alkoxy substituierte $C_1$-$C_6$-Alkyl-, $C_2$-$C_6$-Alkenyl-, $C_2$-$C_6$-Alkinyl- oder Phenyl-$C_1$-$C_4$-alkylgruppe oder eine gegebenenfalls durch Halogen, Nitro oder $C_1$-$C_4$-Alkyl substituierte Phenylgruppe,

$R_{11}$ und $R_{12}$, die gleich oder verschieden sind, ein Wasserstoffatom, einen gegebenenfalls ein- oder mehrfach, gleich oder verschieden durch Halogen, Hydroxy oder $C_1$-$C_4$-Alkoxy substituierten $C_1$-$C_6$-Alkyl-, $C_2$-$C_6$-Alkenyl-oder $C_2$-$C_6$-Alkinylrest oder

$R_{11}$ und $R_{12}$ gemeinsam mit dem benachbarten Stickstoffatom die Pyrrolidinyl-, Piperidino- oder Morpholinogruppe,

X ein Sauerstoff- oder Schwefelatom und

n 0, 1 oder 2

bedeuten.

2. 6,7-Dihydro-pyrazolo[1,5-a][1,3,5]triazin-2-sulfonsäureamide gemäß Anspruch 1, dadurch gekennzeichnet, daß in der allgemeinen Formel I

Ar eine Phenylgruppe der allgemeinen Formel

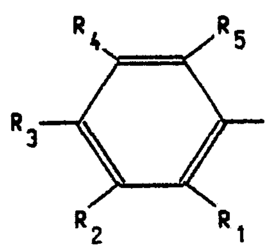

$R_1$ und $R_5$, die gleich oder verschieden sind, ein Halogenatom, eine Methylgruppe, eine Trifluormethylgruppe, eine Nitrogruppe, eine Methoxygruppe oder eine Methoxycarbonylgruppe,

$R_2$, $R_3$ und $R_4$, die gleich oder verschieden sind, ein Wasserstoffatom, ein Halogenatom, eine Trifluormethyl- oder eine $C_1$-$C_4$-Alkylgruppe,

$R_6$ ein Wasserstoffatom, ein einwertiges Metalläquivalent oder eine $C_1$-$C_4$-Acylgruppe,

$R_7$ und $R_8$, die gleich oder verschieden sind, ein Wasserstoffatom, eine $C_1$-$C_4$-Acylgruppe, eine $C_1$-$C_4$-Alkylgruppe, eine $C_2$-$C_4$-Alkenylgruppe oder eine Phenylgruppe,

$R_9$ ein Wasserstoffatom, eine $C_1$-$C_4$-Acylgruppe, eine Gruppe $R_{10}$-O-CO-, eine Aminocarbonylgruppe $R_{11}R_{12}$N-CO-, eine Sulfonylgruppe $R_{10}$-$SO_2$-, eine Cyanogruppe oder eine Nitrogruppe und

X ein Sauerstoff- oder Schwefelatom

bedeuten.

3. Verfahren zur Herstellung von 6,7-Dihydro-pyrazolo[1,5-a][1,3,5]triazin-2-sulfonsäureamiden der allgemeinen Formel I, dadurch gekennzeichnet, daß man

A) Amine der allgemeinen Formel II,

Ar-NH-$R_6$ (II) ,

in der Ar die oben angegebene Bedeutung hat und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Sulfonsäurechlorid der allgemeinen Formel III

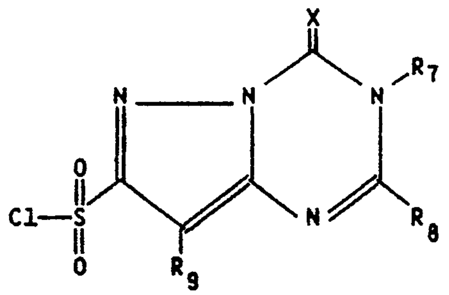

(III) ,

in der $R_7$, $R_8$, $R_9$ und X die oben angegebenen Bedeutungen haben, in einem geeigneten Lösungsmittel in Gegenwart eines Säureakzeptors umsetzt, oder

B) eine Verbindung der allgemeinen Formel IV

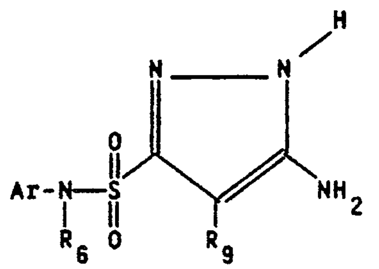

(IV) ,

in der Ar und $R_9$ die oben angegebenen Bedeutungen haben und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Isocyanat oder Isothiocyanat der Formel V

$R_7$-NCX (V) ,

in der $R_7$ und X die oben angegebenen Bedeutungen haben, in einem geeigneten Lösungsmittel gegebenenfalls in Gegenwart eines Säureakzeptors beziehungsweise Katalysators umsetzt und die entstandenen Verbindungen der allgemeinen Formel VI

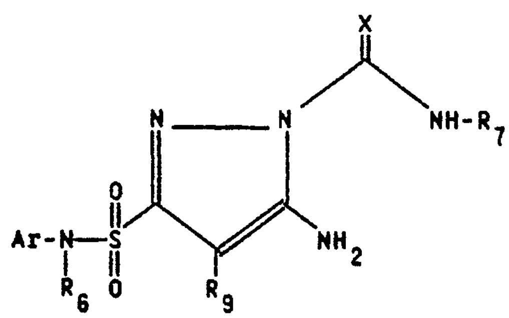

(VI) ,

in der Ar, $R_7$, $R_9$ und X die oben angegebenen Bedeutungen haben und $R_6$ ein Wasserstoffatom, eine $C_1$-$C_6$-Alkyl-, $C_2$-$C_6$-Alkenyl- oder $C_2$-$C_6$-Alkinylgruppe bedeutet, mit einem Orthoester der Formel VII

$R_8$-C($OR_{10}$)$_3$ (VII),

in der $R_{10}$ die oben angegebene Bedeutung hat, aber nicht für ein Wasserstoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl-oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest oder einen durch $R_1$, $R_2$, $R_3$, $R_4$ und $R_5$ substituierten Phenylrest bedeutet, in einem geeigneten Lösungsmittel, das auch der Orthoester selbst sein kann, umsetzt, oder

C) eine Verbindung der allgemeinen Formel IV

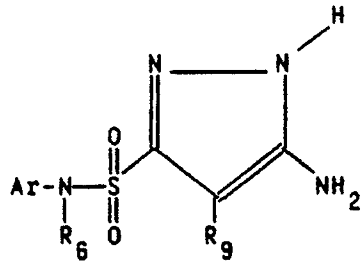

(IV) ,

in der Ar und $R_9$ die oben angegebenen Bedeutungen haben und $R_6$ ein Wasserstoffatom oder eine $C_1$-$C_6$-Alkyl-, $C_2$-$C_6$-Alkenyl- oder $C_2$-$C_6$-Alkinylgruppe bedeutet, mit einem Orthoester der allgemeinen Formel VII

$R_8$-C($OR_{10}$)$_3$ (VII) ,

in der $R_{10}$ die oben angegebene Bedeutung hat, aber nicht für ein Wasser stoffatom steht, und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl-oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl- oder Phenyl-$C_2$-$C_6$-alkinylrest oder einen durch $R_1$, $R_2$, $R_3$, $R_4$ und $R_5$ substituierten Phenylrest bedeutet, zu Verbindungen der allgemeinen Formel VIII

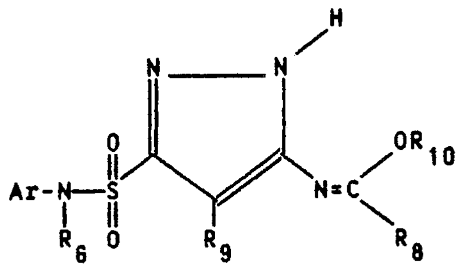

(VIII) ,

in der Ar, $R_9$ und $R_{10}$ die oben angegebenen Bedeutungen haben, $R_{10}$ aber nicht für ein Wasserstoffatom steht, $R_6$ ein Wasserstoffatom oder eine $C_1$-$C_6$-Alkyl-, $C_2$-$C_6$-Alkenyl- oder $C_2$-$C_6$-Alkinylgruppe und $R_8$ ein Wasserstoffatom, einen gegebenenfalls durch Halogen und/oder $C_1$-$C_4$-Alkoxy substituierten $C_1$-$C_6$-Alkyl-, $C_2$-$C_6$-Alkenyl- oder $C_2$-$C_6$-Alkinylrest, einen gegebenenfalls durch Halogen, $C_1$-$C_4$-Alkyl, $C_1$-$C_4$-Alkoxy, $C_1$-$C_4$-Alkoxy-$C_1$-$C_4$-alkyl, Halogen-$C_1$-$C_4$-alkyl substituierten Phenyl-$C_1$-$C_6$-alkyl-, Phenyl-$C_2$-$C_6$-alkenyl-oder Phenyl-$C_2$-$C_6$-alkinylrest oder einen durch $R_1$, $R_2$, $R_3$, $R_4$ und $R_5$ substituierten Phenylrest bedeuten, in einem geeigneten Lösungsmittel, das auch der Orthoester selbst sein kann, umsetzt und dann die Verbindungen der allgemeinen Formel VIII mit einem Isocyanat oder Isothiocyanat der Formel V

$R_7$-NCX (V)

in der $R_7$ und X die oben angegebenen Bedeutungen haben, in einem geeigneten Lösungsmittel gegebenenfalls in Gegenwart eines Säureakzeptors beziehungsweise Katalysators umsetzt, oder

D) eine Verbindung der allgemeinen Formel IX

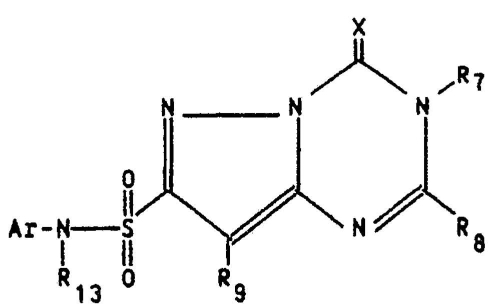

(IX) ,

in der Ar, $R_7$, $R_8$, $R_9$ und X die oben angegebenen Bedeutungen haben und $R_{13}$ ein Wasserstoffatom oder ein einwertiges Metalläquivalent bedeutet, mit Verbindungen der allgemeinen Formel X

$R_{10}$-Hal (X) ,

oder der allgemeinen Formel XI

$R_{10}$-CO-Hal (XI) ,

in denen $R_{10}$ die oben genannte Bedeutung hat, aber nicht für ein Wasserstoffatom steht, und Hal Chlor oder Brom bedeutet, oder der allgemeinen Formel XII

$R_{10}$-CO-O-CO-$R_{10}$ (XII) ,

in der $R_{10}$ die oben genannte Bedeutung hat, in einem geeigneten Lösungsmittel umsetzt, oder

E) eine Verbindung der allgemeinen Formel XIII

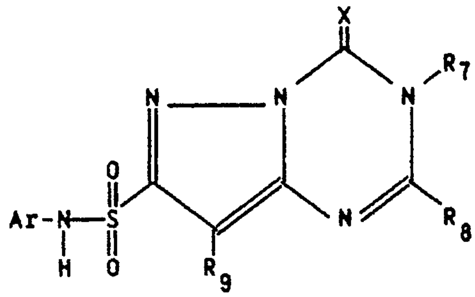

(XIII) ,

in der Ar, $R_7$, $R_8$, $R_9$ und X die oben angegebenen Bedeutungen haben, mit einer Verbindung der allgemeinen Formel XIV

M - Y (XIV) ,

in der M ein einwertiges Metalläquivalent und Y ein Wasserstoffatom, eine Hydroxy-, Niederalkyl-, Niederalkoxy- oder Aminogruppe bedeutet, in einem Lösungsmittel umsetzt.

4. Mittel mit herbizider und pflanzenwuchsregulierender Wirkung, gekennzeichnet durch einen Gehalt an mindestens einer Verbindung gemäß den Ansprüchen 1 und 2.

5. Verwendung von Verbindungen gemäß den Ansprüchen 1 und 2 zur Bekämpfung monokotyler und dikotyler Unkrautarten in landwirtschaftlichen Hauptkulturen.

6. Verfahren zur Herstellung von Mitteln mit herbizider und pflanzenwuchsregulierender Wirkung, dadurch gekennzeichnet, daß man Verbindungen der allgemeinen Formel I gemäß den Ansprüchen 1 und 2 mit Träger- und/oder Hilfsstoffen vermischt.

Europäisches Patentamt

# EUROPÄISCHER RECHERCHENBERICHT

Nummer der Anmeldung

EP 88110245.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 004 171 (IMPERIAL CHEMICAL INDUSTRIES LIMITED)<br>* Ansprüche 1,5,9 *<br>-- | 1-6 | C 07 D 487/04<br>A 01 N 43/90//<br>(C 07 D 487/04<br>C 07 D 251:00<br>C 07 D 231:00) |
| D,A | EP - A2 - 0 142 152 (THE DOW CHEMICAL COMPANY)<br>* Ansprüche 1,43,67 *<br>-- | 1-6 | |
| A | GB - A - 951 652 (W.BROABENT et al.)<br>* Ansprüche 1,5 *<br>-- | 1-3 | |
| D,A | EP - A2 - 0 150 974 (THE DOW CHEMICAL COMPANY)<br>* Ansprüche 1,7,9 *<br>-- | 1-6 | RECHERCHIERTE SACHGEBIETE (Int. Cl.4) |
| P,A | EP - A2 - 0 245 785 (BAYER AG)<br>* Anspruch 1 *<br>---- | 3 | C 07 D 487/00<br>A 01 N 43/00<br>C 07 D 231/00 |

Der vorliegende Recherchenbericht wurde für alle Patentansprüche erstellt.

| Recherchenort | Abschlußdatum der Recherche | Prüfer |
|---|---|---|
| WIEN | 15-09-1988 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82